# EUROPÄISCHE PATENTSCHRIFT

(11) **EP 1 220 656 B1**
(45) Veröffentlichungstag und Bekanntmachung des Hinweises auf die Patenterteilung: **13.08.2003**
(21) Anmeldenummer: 00974391.5
(22) Anmeldetag: 13.10.2000
(51) Int. Cl.: A61K 9/02, A61K 47/10

(54) **FORMKÖRPER MIT EINER AMPHIPHILEN ÄUSSEREN PHASE UND EINER LIPOPHILEN INNEREN PHASE**
SHAPED BODY COMPRISING AN AMPHIPHILIC OUTER PHASE AND A LIPOPHILIC INNER PHASE
CORPS MOULES CONTENANT UNE PHASE EXTERNE AMPHIPHILE ET UNE PHASE INTERNE LIPOPHILE

(30) Priorität: 15.10.1999 DE 19949897
(43) Veröffentlichungstag der Anmeldung: 10.07.2002
(73) Patentinhaber: Rogasch, Rainer, 34513 Waldeck (DE); Fisseler, Friedrich, 34516 Vöhl (DE); Schmieding, Marc, 34497 Korbach (DE)
(72) Erfinder: Rogasch, Rainer, 34513 Waldeck (DE); Fisseler, Friedrich, 34516 Vöhl (DE); Schmieding, Marc, 34497 Korbach (DE)
(74) Vertreter: Olbricht, Karl Heinrich, Dipl.-Phys.
(86) Internationale Anmeldenummer: EP0010113
(87) Internationale Veröffentlichungsnummer: WO01028589

(56) Entgegenhaltungen:
- EP-A- 0 094 513
- EP-A- 0 103 995
- EP-A- 0 709 085
- EP-A- 0 748 630
- WO-A-00/03660
- WO-A-96/14059
- GB-A- 887 872
- US-A- 4 344 968
- US-A- 4 347 237
- US-A- 4 874 774
- US-A- 5 393 738
- US-A- 5 700 486
- REGDON G ET AL: "FORMULATION AND IN VITRO STUDY OF THEOPHYLLNE CONTAINING SUPPOSITORIES" PHARMAZIE,DD,VEB VERLAG VOLK UND GESUNDHEIT. BERLIN, Bd. 51, Nr. 5, 1. Mai 1996 (1996-05-01), Seiten 347-352, XP000582508 ISSN: 0031-7144

## Beschreibung

Die Erfindung betrifft einen Formkörper zur medizinischen Anwendung in Körperund/oder Wundhöhlungen am menschlichen oder tierischen Körper gemäß dem Oberbegriff von Anspruch 1, sowie ein Verfahren zum Herstellen solcher Formkörper gemäß dem Oberbegriff des Anspruchs 13 und eine Verwendung derselben gemäß Anspruch 18 und 19.

Pharmazeutische oder medizinische Stifte (Formkörper) sind eine alte Arzneiform, die gewöhnlich durch Ausgießen und Aushärten geeigneter pharmazeutischer Hilfsstoffe in entsprechende Gießformen gewonnen wird. Eine häufig verwendete, spezielle Form medizinischer Stifte stellen Suppositorien zur analen oder rektalen Applikation dar, die in der Regel auf der Basis von Triglyceriden hergestellt werden. Da Triglyceride (Fette) zu Sprödigkeit neigen, ist es mit diesen Materialien kaum möglich, Stifte mit einem Durchmesser von weniger als 4 mm und Längen von mehr als 20 mm dauerhaft bruchstabil herzustellen.

Um dem zu begegnen wäre es möglich, reine Kunststoff/Wirkstoffgemische zu verwenden und zu Stiften beliebiger Durchmesser zu extrudieren. Derartige Stifte hätten zwar eine ausreichende Bruchfestigkeit sowie eine hohe Elastizität und damit die gewünschten Materialeigenschaften, sie zeigen allerdings gravierende Nachteile hinsichtlich der Verträglichkeit in physiologischen Kompartimenten, da Kunststoffe eine nur geringe oder keine Abbaubarkeit aufweisen. Wird das Trägermaterial hingegen zu schnell abgebaut, ist meist keine ausreichende Versorgung des Zielkompartiments mit dem erforderlichen Arzneistoff möglich, was die Wundheilung beeinträchtigen kann.

Diesen Hindernissen wird in der Patentanmeldung EP-A1-064514 dadurch begegnet, daß Polyethylenglykole als amphiphile Agenzien beigemischt werden. Trotz der gewonnenen Elastizität der gebildeten Formkörper ist es nicht möglich, große Gewichtsprozentanteile von Lipiden oder Fettsäuren im Formkörper dispers zu stabilisieren und dadurch die Abgabegeschwindigkeit verschieden polarer pharmazeutischer Wirkstoffe in einem weiten Bereich zu variieren.

In EP-A1-0 748 630 gelingt es, Urinastatin einthaltende Suppositorien herzustellen, die 70 bis 99,5 Gew.-% an lipophiler, innerer Phase enthalten. Man mischt hierzu das Hartfett Witepsol W35® mit bis zu 5 Gewichtsanteilen eines amphiphilen Polymers beispielsweise eines Poloxamers, und erwärmt bis zur Schmelze. Dieser Schmelze wird bei 35 bis 45° C eine wässrige Phase zugesetzt, die den Wirkstoff Ulinastatin enthält. Es entsteht eine Mischung, die zu einer Emulsion weiterverarbeitet und zum Aushärten in Kapselhüllen für Zäpfchen gefüllt wird. Die so erhaltenen Formkörper sind zum Einführen in die menschliche Scheide gedacht, in der sie sich bei Körpertemperatur schnell auflösen. Zu einer verlangsamten Abgabe des Wirkstoffs kann es demnach trotz eines hohen Gewichtsanteils an Lipiden oder Fettsäuren nicht kommen.

Auch die in GB-PS-0 887 872 beschriebenen pharmazeutischen Zubereitungen zerfallen bereits zwei Stunden nach Einführen in eine Körperhöhlung vollständig. Sie bestehen zu 33 bis 48 Gew.-% aus Polyethylenglykolen unterschiedlicher Molmassen und enthalten 26 bis 40 Gew.-% eines festen, größtenteils wasserlöslichen Trägermaterials. Um hierin 10 bis 12 Gewichtsanteile Walrat als lipophile, innere Phase sowie Wirkstoffe dispers zu stabilisieren, werden u.a. Poloxamere mit Gewichtsanteilen von 4 bis 10 % als Detergenzien zugegeben. Man kühlt die erhaltene disperse Masse ab und lagert sie unterhalb Raumtemperatur um sie danach zu extrudieren. Die so gewonnene Zubereitung behält ex vivo selbst bei einer Temperatur von 47 °C wenigstens einen Monat lang ihre Form und ihre Steifheit. Sie wird demnach kaum biegeelastisch sein und bei mechanischer Beanspruchung leicht zerbrechen. Deshalb kann auch aus diesem Grung eine kontinuierlich gleichmäßige und retardierte Wirkstoffliberation im gesamten Zielkompartiment nicht erreicht werden.

Ziel der Erfindung ist es, diese und weitere Nachteile des Standes der Technik zu vermeiden und Formkörper zur medizinischen Anwendung in Körper und/oder Wundhöhlungen am menschlichen oder tierischen Körper zu schaffen, die eine hohe Elastizität sowie eine hohe Bruchfestigkeit aufweisen und die enthaltenen Wirkstoffe bzw. Wirkstoffkombinationen über einen möglichst langen Zeitraum konstant abgeben.

Hauptmerkmale der Erfindung sind in den kennzeichnenden Teilen der Ansprüche 1 und 13 sowie 18 und 19 angegeben. Ausgestaltungen sind Gegenstand der Ansprüche 2 bis 12 und 14 bis 17.

Bei einem Formkörper zur medizinischen Anwendung in Körper- und/oder Wundhöhlungen am menschlichen oder tierischen Körper, mit wenigstens einer amphiphilen äußeren Phase, beispielsweise einem in Wasser dispergierbaren Polymer, mit wenigstens einer lipophilen Komponente als innerer Phase sowie mit wenigstens einem pharmazeutisch wirksamen wasser- oder fettlöslichen Stoff, wobei die äußere Phase ein biologisch neutrales Oligopolymer eines Polyethers, beispielsweise ein Copolymer ist, sieht die Erfindung gemäß Anspruch 1 vor, daß die äußere Phase ein Poloxamer ist, beispielsweise Poloxamer 124, Poloxamer 188, Poloxamer 237 Poloxamer 338 oder Poloxamer 407, daß der Anteil der äußeren Phase wenigstens 40 Gew.-% und der Anteil der inneren Phase bis zu 50 Gew.-% beträgt, wobei die äußere Phase, die innere Phase und der pharmazeutisch wirksame Stoff als fest-disperse Masse nach definierter Reifung bzw. Lagerung bei konstanter. Temperatur unter Druckverflüssigung geformt ist und wobei der geformte Körper einen Durchmesser bis hinab zu 2 min und eine Länge bis zu 100 mm aufweist.

Dadurch erhält der Formkörper eine außerordentlich hohe Elastizität und Bruchfestigkeit, was insbesondere beim Einführen in eine Körper- oder Wundhöhle (Cavität) besonders wichtig ist. Ferner ist gewährleistet, relativ hohe Gewichtsprozentanteile der inneren lipophilen Phase dispers zu stabilisieren. Der Formkörper ist in der Lage, über viele Stunden im Zielkompartiment zu verweilen und den Gehalt an (pharmazeutischen) Wirkstoffen über lange Zeit (retardiert) an die Umgebung abzugeben. Die Heilwirkungen sind außerordentlich gut. Die Verwendung von Poloxameren stellt sicher, daß keine unerwünschten Wechselwirkungen mit der Umgebung auftreten.

Besagte Stoffe mit der für sie typischen Formel HO-(CH₂-CH₂-O)ₐ-(CH₃-CH-CH₂-O)_{b}-(CH₂-CH₂-O)ₐ-H mit a = 2 bis 130 und b = 15 bis 67 zeichnen sich in Bezug auf die Erfindung besonders dadurch aus, daß lipophile Anteile wie z.B. Bienenwachs oder Triglyceride in hohen Konzentrationen bis hin zu 50 Gew.-% homogen dispers mit ihnen vermischbar sind, ohne daß der Formkörper die für das Einführen in die Cavität erforderliche Elastizität bzw. Bruchfestigkeit verliert. Dies gilt auch für die beanspruchten Verbindungen Poloxamer 124, Poloxamer 188, Poloxamer 237, Poloxamer 338 oder Poloxamer 407.

Weil nach der Erfindung selbst Formkörper mit einem zylindrischen Korpus von 2 mm Durchmesser und einer Länge bis zu 100 mm diese vorteilhaften Eigenschaften aufweisen, eignen sie sich zur Applikation in Wund- und Körperhöhlen an Mensch oder Tier.

In der Ausgestaltung von Anspruch 2 verwendet man als lipophile Komponente ein Wachs, insbesondere einen Ester einer langkettigen Fettsäure mit einem langkettigen Fettalkohol, beispielsweise officinelles Bienenwachs EuAB 98 oder gebleichtes Bienenwachs EuAB 98 oder man verwendet als lipophile Komponente einen Fettalkohol bzw. ein Gemisch von Fettalkoholen, beispielsweise Stearylalkohol oder Cetyl-/Stearylalkohol.

Gemäß Anspruch 3 kann die lipophile Komponente eine Triglyceridmischung sein, beispielsweise höher schmelzende Suppositorien-Massen mit einem Schmelzpunkt über 40 °C. Oder man setzt laut Anspruch 4 als lipophile Komponente ein langkettiges Kohlenwasserstoffgemisch ein, beispielsweise höher schmelzende Paraffinmassen mit einem Schmelzpunkt über 40 °C. In Betracht kommen aber auch Ozokeride (Harlparaffine) oder Mischungen pharmazeutisch gebräuchlicher Fettalkohole (Cetyl/Stearylalkohole) als retardierende innere Matrix, die zur verzögerten Freisetzung der dem Formkörper zugesetzten Wirkstoffe am Wirkort dienen. Zur Fixierung sehr unpolarer Wirkstoffe ist die Verwendung von Gemischen langkettiger Kohlenwasserstoffe sehr vorteilhaft.

Zur Variation der Verweilzeit des Formkörpers in der Cavität trägt auch Anspruch 5 bei, wonach der Anteil der lipophilen Komponente zwischen 25 und 50 Gew.-%, bevorzugt zwischen 40 und 50 Gew.-%, und der Anteil der außeren Phase zwischen 40 und 70 Gew.-% bevorzugt zwischen 48 und 52 Gew.-% liegt. Der Anteil der lipophilen Phase, welche in der Regel die pharmazeutischen Wirkstoffe enthält, ist damit relativ groß gegenüber der äußeren Phase. Die Wundheilung wird durch die retardierte Wirkstoffabgabe optimal unterstützt. Dennoch besitzt der Formkörper stets eine ausreichende Formstabilität, weshalb er beim Einführen in die Cavität nicht zerbricht.

Die Auswahl möglicher Wirkstoffe und/oder Wirkstoffkombinationen, beispielsweise antiinfektiöse, antimikrobiell, fungistatisch oder fungizid, virustatisch oder viruzid wirkende pharmazeutische Stoffe allein oder in Kombination mit steroidalen Antiphlogistika, Lokalanästhetika oder Anästhetika, geht aus den Ansprüchen 6 bis 10 hervor. Der Anteil der Wirkstoffe oder Wirkstoffkombination sollte 10 Gew.-% nicht übersteigen. Als Wirkstoff kann ein Antibiotikum, z. B. Benzylpenicillin-Procain oder Gentamicin, ein Antiseptikum, ein Antimykotikum und/oder ein Virustatikum, z. B, Aciciovir enthalten sein. Oder man verwendet ein Glucocortikoid, z. B. ein Hydrocortison, dessen Ester, ein Betamethason, dessen Ester oder ein Triamcinolonacctonid. Besonderes gute Heilwirkungen werden erzielt, wenn als Wirkstoff PVP-Jod enthalten ist. Je nach Anwendungsgebiet kann auch Metronidazol eingesetzt werden. Auch sind im Einzelfall Höherdosierungen denkbar.

Die Geometrie der erfindungsgemäßen Formkörper kann laut Anspruch 11 dadurch gekennzeichnet sein, daß man einen zylindrischen, kegelförmigen oder walzenförmigen Stift ausbildet. Beispielsweise kann der Formkörper einen zylindrischen Korpus von 2 bis 10 mm Durchmesser und einer Länge von 40 bis 100 mm aufweisen, um zur Applikation in Wund- und Körperhöhlen an Mensch oder Tier geeignet zu sein. Ferner kann der Formkörper gemäß Anspruch 12 eine Zäpfchen- oder Torpedoform oder eine Tablettenform aufweisen. Möglich ist aber auch die Herstellung in Suppositorien-Form (Zäpfchen-, Torpedoform o.dgl.) oder Vaginal-Globuli-Form (Vaginaltabletten). Weitere Geometrien sind realisierbar und allein abhängig vom jeweiligen Einsatzzweck.

Gegenstand des Patents ist ferner ein Verfahren zur Herstellung der erfindungsgemäßen Formkörper gemäß dem unabhängigen Anspruch 13. Dieses ist dadurch gekennzeichnet, daß wenigstens eine hydrophile äußere Phase, beispielsweise ein wasserlösliches oder in Wasser dispergierbares Polymer (z.B. Polyether bzw. Polyester verschiedener Molekulargewichte und Zusammensetzungen oder Emulgatoren verschiedener Molekulargewichte) mit wenigstens einer lipophilen Komponente (z.B. Wachse, Triglyceride o.dgl.) als innerer Phase zu einer Schmelze aufgeschmolzen wird, wobei die Schmelze mit wenigstens einem Wirkstoff oder einer Wirkstoffkombination versetzt und unter konstanten Temperaturbedingungen mechanisch zu einer Masse dispergiert wird. Diese wird in einen Behälter, ein Gefäß o.dgl. ausgegossen, darin mit einem konstanten Temperaturgradienten von wenigstens 5 °C/min abgekühlt und anschließend mindestens 4 Stunden bei konstanter Temperatur zwischengelagert. Anschließend wird die dadurch gewonnene Masse auf eine Temperatur, die 5 bis 10 °C unterhalb des Erweichungspunktes liegt, erwärmt, durch Druck oder Scherung plastisch fließend gemacht und zu einem vorgebbaren Formkörper verpreßt, wobei der Arbeitsdruck 50 bar nicht unterschreitet, beispielsweise mindestens 100 bar beträgt und wobei die Arbeitstemperatur unterhalb der Erweichungstemperatur der Masse liegt. Durch dieses Verfahren wird verhindert, daß die einzelnen Komponenten ausflocken oder auskristallisieren und der Formkörper dadurch spröde wird.

Gemäß Anspruch 14 wird der bzw. die Wirkstoffe oder die Wirkstoffkombination einem aufgeschmolzenen, niedrig-molekularen Polymer zugesetzt, wobei diese Vormischung dispergiert und der Schmelze beigemischt wird, was sich günstig auf die gesamte Verfahrensführung auswirkt.

Zweckmäßig wird die Masse nach Anspruch 15 bei konstanter Temperatur und unter definierbarem Arbeitsdruck zu fest-dispersen Formkörpern verpreßt, wobei man bevorzugt eine Strangpresse verwendet, in der die Masse gemäß Anspruch 16 zu Strängen von 2 bis 10 mm Durchmesser verpreßt wird, die zu Längen von 40 bis 100 mm zerteilt werden.

Die nach dem erfindungsgemäßen Verfahren hergestellten zylindrischen Formkörper (Stifte, styli medicinalis) zeigen gegenüber solchen, die durch bloßes Aufschmelzen und anschließendes Ausgießen in Gießformen hergestellt worden sind, eine außerordentlich hohe Elastizität und Bruchfestigkeit.

Eine bedeutsame Weiterbildung der Erfindung geht aus Anspruch 17 hervor. Danach werden bei einem Verfahren zur Herstellung von gepreßten Formkörpern, welche einen hohen Gehalt an niedrig schmelzenden hydrophoben Bestandteilen besitzen, sowie zur Herstellung von fließfähigen oder rieselfähigen tablettierbaren Mischungen, welche einen hohen Gehalt an niedrig schmelzenden hydrophoben Bestandteilen aufweisen, erfindungsgemäß kleine Perlen einer abgekühlten Schmelze verpreßt, kleine Kügelchen pulverisiert und verpreßt oder ein abgekühltes Pulver in Kapseln verbracht.

Eine pharmazeutische Zubereitung ist gemäß Anspruch 18 gekennzeichnet durch eine fest-disperse Fett-in-Polymer-Dispersion oder eine Fett-in-Detergens-Dispersion.

Ein noch anderer Gegenstand der Erfindung ist schließlich die in Anspruch 19 formulierte Verwendung von PVP-Jod zusammen mit einem Poloxamer zur Herstellung eines bis 100 mm langen und bis 2 mm dünnen, bruchstabilen und biegeelastischen Formkörpers zur Behandlung von Wunden in milchproduzierenden. Organen landwirtschaftlicher Nutztiere.

Weitere Merkmale, Einzelheiten und Vorteile der Erfindung ergeben sich aus dem Wortlaut der Ansprüche sowie aus der folgenden Beschreibung von Ausführungsbeispielen.

Ein erfindungsgemäßer Formkörper besitzt eine amphiphile äußere Phase, beispielsweise ein biologisch neutrales Oligopolymer eines Polyethers, eine lipophile innere Komponente, beispielsweise ein officinelles Bienenwachs EuAB 98 oder gebleichtes Bienenwachs EuAB 98, sowie einen wasser- oder fettlöslichen Wirkstoff wie z.B. PVP-Jod. Die äußere Phase und die darin dispergierte innere lipophile Phase bilden ein disperses "Fett-in-Polymer-System", welches die darin gelösten medizinischen Wirkstoffe retardiert an die Umgebung abgeben kann.

Die Formkörper sind bevorzugt Stifte mit Durchmessern von 2 bis 19 mm, vorzugsweise 2 bis 10 mm, und Längen von 40 bis 100 mm, die aufgrund der neuartigen Zusammensetzung durch hohe Elastizität und Biegefestigkeit gekennzeichnet sind. Sie finden als Arzneimittel Anwendung bei der Behandlung von Krankheiten und Verletzungen. Insbesondere können sie problemlos in Wundhöhlungen eingeführt werden, wo sich die gelösten Wirkstoffe freisetzen.

Die Wirkstoffabgabegeschwindigkeit hängt von der Menge an lipophiler Phase im Formkörper ab. Sie ist um so niedriger, je mehr lipophile Phase eingesetzt werden kann. Heteropolymere und Heteroblockpolymere (z. B. Copolymere) in einer Konzentration bis zu 70 Gew.-% (vorteilhaft sind 48 bis 52 Gew.-%) als äußere Phase gestatten ein Einbringen der lipophilen inneren Phase in Konzentrationen bis hin zu 50 Gew.-%. Unter speziellen Bedingungen sind noch höhere Gewichtsanteile der lipophilen Phase verwendbar. Wichtige Vertreter dieser heteropolymeren Alkoxamere sind die Polyether Poloxamer 124, Poloxamer 188, Poloxamer 237, Poloxamer 338 und Poloxamer 407. Allgemein ist es auch möglich, etherartige Polymere mit einem Molekulargewicht von 300 g/mol bis 30.000 g/mol für die Formulierung einzusetzen.

Zudem wird die Abgabegeschwindigkeit des Wirkstoffs von der Verweildauer des Formkörpers und seinem Zerfall im Zielkompartiment, also vom ausgewählten Polymer für die äußere Phase und dessen mechanischer und chemischer Beständigkeit beeinflußt. In physiologischem Milieu labile Polymere werden entweder durch Oberflächenerosion oder durch hydrolytische Spaltung in einem von der Polymerkomposition abhängigen Zeitintervall zu Monomeren abgebaut, welche vom Organismus metabolisierbar sind. Die Verwendung hydrolytisch spaltbarer Polymere erlaubt, die Abgabegeschwindigkeit der pharmazeutisch aktiven Substanz nicht nur über die Verweildauer des Formkörpers im Organismus oder über die Konzentration an innerer Phase einzustellen, sondern sie auch über die Stabilität des Formkörpers zu beeinflussen. Gerade biologisch unbedenkliche, abbaubare Polyester, Polyamidester, Polyanhydride oder Polycarbonate in den verschiedensten Ausführungsformen, so z. B. die Kombination von D- und L-Lactiden, bieten vielfältigste Möglichkeiten Freisetzungskinetiken wirkaktiver Spezies zu steuern. Weiterhin sind beispielsweise die biologisch abbaubaren Polyester Poly(dioxanon-co-ε-caprolacton), Poly(lactid-co-glykolid), Poly(glykolid-co-trimethylencarbonat), Poly(hydroxybutyrat-co-hydroxyvalerat), Poly(trimethylencarbonat-co-ε-caprolacton), Poly(DL-lactid-co-ε-caprolacton) Poly(glykolid-co-ε-caprolacton) oder das Terpolymer Poly(glykolid-co-trimethylencarbonat-co-p-dioxanon) für dieses Aufgabenfeld sehr geeignet. Erwähnt werden müssen in diesem Zusammenhang auch die Verbindungen Polyglycolid, Poly(ε-caprolacton), Polydioxanon, Polytrimethylencarbonat, Polyhydroxybutyrat und Polyhydroxyvalerat.

Wird eine sehr langsame Freisetzung des Medikaments gewünscht und/oder soll der Formkörper sich im Organismus durch Schmelzen oder Zerfallen nur sehr langsam oder überhaupt nicht zersetzen, muß die Konzentration an innerer Phase sehr hoch und das eingesetzte Polymer gegen Hydrolyse relativ beständig sein. Neben den Polyethern sind hier bevorzugt Polyamide und polymere Strukturen, in denen durch Peptidbindung verknüpfte D-Aminosäurebereiche vorhanden sind, einzusetzen.

Geht es darum, sich aggregierende Wirkstoffe oder schlecht solubilisier- und emulgierbare pharmazeutische Substanzen in fein verteilter Form in einen Formkörper zu verbringen, so ist es sinnvoll, als äußere Phase neutral geladene oder nicht ionische, biologisch unschädliche Detergenzien einzusetzen. Dabei ist es günstig, solche Vertreter zu wählen, die eine CMC größer 6 haben, da es sonst zu übermäßigem Schäumen im Gewebekompartiment und zur teilweisen Lyse von Zellen kommen würde. Beispielsweise sind N-Octylglucosid, Dodecylmaltosid oder CHAPS verwendbar.

Speziell bei der Herstellung von Tabletten und Stäbchen ist die Verwendung von Polyethylenglykolen besonders vorteilhaft. Werden PEG 1000, PEG 1500, PEG 2000 und PEG 4000 und weiterhin PEG 200 oder PEG 400 zur Bildung der äußeren Phase eingesetzt, so können die erhaltenen Dispersionen nach Reifung nicht nur direkt zu Presslingen, sondern auch zu kleinen Kügelchen oder zu Pulver weiterverarbeitet werden. Das Pulver kann in Kapseln verfüllt oder zu Tabletten gepreßt werden. Aus den Perlen ist ebenfalls eine Tablette mit jedoch anderer Feinverteilung herstellbar. PEG 200 und PEG 400 haben überdies den Vorteil, daß man sie als niedermolekulare Polyethylenglykole zur Benetzung oder Auflösung der wirkaktiven Spezies verwenden kann. Dies geschieht, wenn nötig, vor dem eigentlichen Dispergierprozeß.

Die äußere Phase läßt sich im Bedarfstall auch aus Mischpolymeren der genannten Polymerklassen oder aus Emulgator/Polymermischungen aufbauen. Dies ist dann von besonderem Nutzen, wenn eine Wirkspezies zum einen nur im Inneren von Mizellen feinverteilt in den Formkörper einzubringen ist und zum anderen diese nur langsam wieder an die Umgebung abgegeben werden soll

Die medizinische Wirkung der erfindungsgemäßen Formkörper läßt sich wie folgt beschreiben:

Durch die topische Anwendung (beispielsweise zur Wunddesinfektion, bei der Versorgung von Bißwunden, bei der Applikation in Zitzen von milchgebenden Nutztieren u.dgl.) von (Antiinfektiosa, Chemotherapeutika) antimikrobiell, fungiziden oder viruziden pharmazeutischen Wirkstoffen mittels der Formkörper wird eine bakteriostatische oder bakterizide, viruzide oder fungizide Wirkung an oder im Zielkompartiment, d.h. der Körperoder Wundhöhle erzielt, die gekennzeichnet ist durch eine hohe, gleichmäßige lokale Wirkstoffkonzentrationen über einen definierten Zeitraum (retardierte Wirkstoffliberation) und gleichzeitig geringe systemische Aufnahme von Wirkstoffen.

Der Zusatz von steroidalen entzündungshemmenden Wirkstoffen (Antiphlogistika) hat abschwellende und verklebungshemmende Wirkung. Der Zusatz von geeigneten Schmerzmitteln oder Lokalanästhetika hemmt zudem den Wundschmerz.

Der unter anderem durch die lipophile innere Phase retardierte Formkörper verbleibt über Stunden am Wirkort und hält so das Lumen des betroffenen Kompartiments offen, was insbesondere bei der Applikation in Zitzen von erheblichem Vorteil ist. Ein Verkleben des betroffenen Gewebes wird zuverlässig vermieden. Die bei Verletzungen auftretende sekundäre Schwellung wird reduziert. Die im Formkörper verwendeten, wasserlöslichen oder wasserdispergierbaren Polymere (vorwiegend Polyether und Polyester, aber auch Emulgatoren und Polyamide) sind zudem sekretaufnehmend und entfalten so eine adjuvante antimikrobielle Wirkung. Sie sind darüber hinaus proliferationsfördernd; sie beschleunigen somit die Wundheilung. Mögliche Zusammensetzungen der Formkörper können wie folgt gewählt werden (BW = Bienenwachs; HC = Hydrocortison):

| | | | | | |
|---|---|---|---|---|---|
| Beispiel 1 < | PVP-JOD | 5,0 g | Beispiel 6 | PVP-JOD | 5,0 g |
| | HC | 1,0 g | | HC | 1,0 g |
| | Poloxamer 188 | 79,0 g | | Poloxamer 188 | 77,5 g |
| | BW | 5,0 g | | BW | 7,5 g |
| | Ethylalkohol 96 % | 10,0 g | | Macrogol 400 | 10,0 g |
| | | | | | |
| Beispiel 2 | PVP-JOD | 5,0 g | Beispiel 7 | PVP-JOD | 5,0 g |
| | HC | 1,0 g | | HC | 1,0 g |
| | Poloxamer 407 | 79,0 g | | Poloxamer 407 | 79,0 g |
| | BW | 5,0 g | | BW | 5,0 g |
| | Propylenglykol | 10,0 g | | Macrogol 400 | 10,0 g |
| | | | | | |
| Beispiel 3 | PVP-JOD | 5,0 g | Beispiel 8 | PVP-JOD | 5,0 g |
| | HC | 1,0 g | | HC | 1,0 g |
| | Macrogol 1500 | 89,0 g | | Poloxamer 407 | 77,5 g |
| | BW | 5,0 g | | BW | 7,5 g |
| | | | | Macrogol 400 | 10,0 g |
| | | | | | |
| Beispiel 4 | PVP-JOD | 5,0 g | Beispiel 9 | PVP-JOD | 5,0 g |
| | HC | 1,0 g | | HC | 1,0 g |
| | Poloxamer 188 | 46,0 g | | Poloxamer 407 | 46,0 g |
| | Witepsol 40 | 40,0 g | | Witepsol 40 | 40,0 g |
| | Ethylalkohol 96 % | 10,0 g | | Ethylalkohol 96 % | 10,0 g |
| | | | | | |
| Beispiel 5 | PVP-JOD | 5,0 g | | | |
| | HC | 1,0 g | | | |
| | Poloxamer 188 | 79,0 g | | | |
| | BW | 5,0 g | | | |
| | Macrogol 400 g | 10,0 g | | | |

Die einzelnen Schritte des erfindungsgemäßen Herstellungsverfahrens können folgendermaßen beschrieben werden: .
1. Die ausgewählten wasserlöslichen oder wasserdispergierbaren Polymere oder Tenside werden mit der lipophilen Komponente (z.B. offizielle Bienenwachse, Fettalkohole, Triglyceride) aufgeschmolzen, wobei sich die Verarbeitungstemperatur nach den Schmelzpunkten der eingesetzten Komponenten richtetet.
2. Der oder die pharmazeutischen Wirkstoffe können gegebenenfalls in einem niedrig-molekularen Macrogol (z.B. PEG 200/400) oder in pharmazeutisch gebräuchlichen Alkoholen gelöst, bzw. dispergiert und anschließend der Schmelze zugemischt oder direkt in der Schmelze dispergiert werden.
   Als pharmazeutische Wirkstoffe können alle für Anwendungen in den Körper- oder Wundhöhlen geeigneten pharmazeutischen Wirkstoffe dienen. Bevorzugt werden dabei folgende Wirkstoffgruppen: Antibiotika, Antiseptika, Antimykotika, Virustatika (Antiinfectiosa, Chemotherapeutika), Antiphlogistika, insbesondere Glucocortikoide und Anaesthetika oder Lokalanaesthetika, die einzeln oder in Kombination miteinander in den einem Fachmann geläufigen Mengen bzw. Konzentrationen zugesetzt werden können.
   Besonders bevorzugte Antibiotika sind Penizilline, insbesondere Benzylpenicillin-Procain und Gentamycin sowie seine chemischen Derivate, bevorzugtes Antiseptikum ist PVP-Jod. Als Glucocortikoide werden bevorzugt Hydrocortison und seine Ester, Betamethason und seine Ester, Dexamethason und seine Ester sowie Triamcinolon (Triamcinolonacctonid) und seine Ester eingesetzt. Als Antiinfektiosum und Antiprotozikon wird bevorzugt Metronidazol, als Virustatikum bevorzugt Aciclovir eingesetzt.
3. Die Mischungen aus 1. und 2. werden, falls erforderlich, zusammengeführt und mechanisch dispergiert, wobei die Schmelztemperatur der eingesetzten Polymere nicht unterschritten werden darf.
4. Die Dispersionsschmelze wird in ein thermostatisierbares Gefäß (z. B. einen Zylinder) ausgegossen und zeitgesteuert auf 15 °C abgekühlt und anschließend mindestens 4 Stunden bei 15 °C zwecks Reifung bzw. Aushärtung gelagert.
5. Nach der Kühl-Lagerungsperiode, wird die so gewonnene wirkstoffhaltige, fest-disperse Masse mittels einer thermostatisierbaren Hochdruck-Strangpresse hydraulisch bei Arbeitstemperaturen, die ca. 5 bis 10 °C unterhalb des Erweichungspunktes der jeweils eingesetzten Mischung liegen müssen, mittels verschiedener Düsen oder Vorsatzformen zu Formkörpern ausgepreßt (ausgezogen), wobei der Arbeitsdruck 100 bar beträgt.

In einer Weiterführung ist das Herstellungsverfahren für die Produktion von Tabletten und Kapseln abgewandelt. Dabei wird die unter Druckvorspannung stehende Dispersion bei einer Temperatur unterhalb des Erweichungspunktes durch Pressen durch eine Lochscheibe in kleine Kügelchen überführt. Diese werden gesiebt und hiernach zu Tabletten verpreßt oder unter Kühlung zu einem Pulver gemahlen. Letzteres wird zu Tabletten gepreßt oder in Kapseln abgefüllt

Durch die Kombination von physiologisch unbedenklichen Polymeren oder Emulgatoren mit verschiedenen pharmazeutisch gebräuchlichen Fetten, Wachsen und Fettalkoholen in einem fest-dispersen System lassen sich die Nachteile des Standes der Technik, insbesondere die Grenzen der alleinigen Verwendung von Polymeren oder Fetten bzw. Wachsen überwinden. Bisher mögliche Höchstkonzentrationen der lipophilen Komponente werden durch die Verwendung von Copolymeren und Blockcopolymeren oder deren oligomerer Erweiterungsformen zu höheren Werten verschoben, was die Abgabegeschwindigkeit der Wirkspezies erheblich beeinflußt. Die erfindungsgemäßen festen Fett-in-Polymer-Dispersionen oder Polymer-in-Fett-Dispersionen weisen die für die Handhabbarkeit und Applizierbarkeit erforderlichen Materialeigenschaften auf und stellen gleichzeitig eine retardierte Arzneiform für die Anwendung in Wund- und Körperhöhlen dar. Die Stifte sind physiologisch gut verträglich und abbaubar.

Die erfindungsgemäßen Verfahrensschritte und deren Abfolge gewährleisten eine hohe und stabile Dispersität der medizinischen Stifte, d.h. der Grad der Zerteilung der Stoffe ineinander und die Grenzflächenverbindung der eingesetzten Stoffe miteinander bzw. untereinander ist gegenüber herkömmlichen Formkörpern außerordentlich hoch. Die medizinischen Stifte bilden eine homogene feste Dispersion, die alle erforderlichen Eigenschaften aufweist.

Die Erfindung ist nicht auf eine der vorbeschriebenen Ausführungsformen beschränkt, sondern in vielfältiger Weise abwandelbar. So kann anstelle eines Fett-in-Polymer-Systems auch die Umkehrung, d.h. ein Polymer-in-Fett-System verwendet werden. Weiterhin können nicht lamellare Phasen und invertierte Systeme bestehend aus Lipiden, Tensiden oder Phospholipiden Anwendung finden.

Man erkennt, daß wasserlösliche oder wasserdispergierbare Polymere, insbesondere aus den Substanzklassen der Polyether, Polyester, Polyamide und/oder Detergenzien, sowie lipophile Stoffe durch Aufschmelzen der Komponenten bei geeigneten Temperaturen und unter Zumischung pharmazeutischer Wirkstoffe mittels mechanischer Verfahren gut dispergieren, d.h. eine innere lipophile Phase wird in einer äußeren hydrophilen Polymerphase stabil zerteilt. Durch gesteuerte Abkühlung, Aushärtung und Lagerung und anschließendes Hochdruck-Pressen oder Schneckenextrudieren bei definierten Temperaturen und Arbeitsdrücken lassen sich neben anderen Formkörpern disperse Polymer-Fettstifte herstellen, die sich durch besondere Bruchfestigkeit und Biegefähigkeit auszeichnen. Für die medizinische Anwendung bieten solche Stifte den Vorteil, daß die zur Herstellung verwendeten Polymere und hydrophoben Stoffe (Wachse, Fettalkohole, Triglyceride etc.) physiologisch und toxikologisch unbedenklich sich und beliebige pharmazeutische Wirkstoffe inkorporiert werden können.

Die Kombination von wasserdispergierbaren Polymeren mit hydrophoben Substanzen bietet den großen Vorteil des verzögerten Abbaues der formstabilen Stifte im physiologischen Zielkompartiment, bedingt durch die innere lipophile Matrix, welche von körpereigenen Flüssigkeiten und Sekreten nur langsam aufgelöst werden kann. Damit einher geht eine verzögerte, aber gleichmäßige Wirkstoffabgabe im Zielkompartiment, die ausreichende therapeutische Wirkspiegel pharmazeutischer Wirkstoffe über längere Zeiträume sicherstellt. Ferner kann durch den sich langsam abbauenden Stiftkörper (Formkörper) ein verletztes Körperlumen erhalten und gegen unerwünschtes Verkleben geschützt werden.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Formkörper zur medizinischen Anwendung in Körper- und/oder Wundhöhlungen am menschlichen oder tierischen Körper, mit wenigstens einer amphiphilen äußeren Phase, beispielsweise einem in Wasser dispergierbaren Polymer, mit wenigstens einer lipophilen Komponente als innerer Phase sowie mit wenigstens einem pharmazeutisch wirksamen wasser- oder fettlöslichen Stoff, wobei die äußere Phase ein biologisch neutrales Oligopolymer eines Polyethers, beispielsweise ein Copolymer ist, **dadurch gekennzeichnet, daß**
a) die äußere Phase ein Poloxamer ist, beispielsweise Poloxamer 124, Poloxamer 188, Poloxamer 237, Poloxamer 338 oder Poloxamer 407,
b) der Anteil der äußeren Phase wenigstens 40 Gew.-% und der Anteil der inneren Phase bis zu 50 Gew.-% beträgt,
c) wobei die äußere Phase, die innere Phase und der pharmazeutisch wirksame Stoff als fest-disperse Masse nach definierter Reifung bzw. Lagerung bei konstanter Temperatur unter Druckverflüssigung geformt ist
d) und wobei der geformte Körper einen Durchmesser bis hinab zu 2 mm und eine Länge bis zu 100 mm aufweist.

2. Formkörper nach Anspruch 1, **dadurch gekennzeichnet, daß** die lipophile Komponente ein Wachs ist, insbesondere ein Ester einer langkettigen Fettsäure mit einem langkettigen Fettalkohol, beispielsweise officinelles Bienenwachs EuAB 98 oder gebleichtes Bienenwachs EuAB 98, oder daß die lipophile Komponente ein Fettalkohol bzw. ein Gemisch von Fettalkoholen ist, beispielsweise Stearylalkohol oder Cetyl-/Stearylalkohol.

3. Formkörper nach einem der Ansprüche 1 oder 2, **dadurch gekennzeichnet, daß** die lipophile Komponente eine Triglyceridmischung ist, beispielsweise höher schmelzende Suppositorien-Massen mit einem Schmelzpunkt über 40 °C.

4. Formkörper nach einem der Ansprüche 1 bis 3, **dadurch gekennzeichnet, daß** die lipophile Komponente ein langkettiges Kohlenwasserstoffgemisch ist, beispielsweise höher schmelzende Paraffinmassen mit einem Schmelzpunkt über 40 °C.

5. Formkörper nach einem der Ansprüche 1 bis 4, **dadurch gekennzeichnet, daß** der Anteil der lipophilen Komponente zwischen 25 und 50 Gew.-%, bevorzugt zwischen 40 und 50 Gew.-%, liegt und daß der Anteil der äußeren Phase 40 bis 70 Gew.-%, bevorzugt zwischen 48 und 52 Gew.-% beträgt.

6. Formkörper nach einem der Ansprüche 1 bis 5, **dadurch gekennzeichnet, daß** der Anteil der Wirkstoffe oder der Wirkstoffkombination 10 Gew.-% nicht übersteigt.

7. Formkörper nach einem der Ansprüche 1 bis 6, **dadurch gekennzeichnet, daß** als Wirkstoff ein Antibiotikum, z. B. Benzylpenicillin-Procain oder Gentamycin, ein Antiseptikum, ein Antimykotikum und/oder ein Virustatikum, z. B, Aciclovir enthalten ist.

8. Formkörper nach einem der Ansprüche 1 bis 7, **dadurch gekennzeichnet, daß** als Wirkstoff ein Glucocortikoid, z. B. ein Hydrocortison, dessen Ester, ein Betamethason, dessen Ester oder ein Triamcinolonacctonid enthalten ist.

9. Formkörper nach einem der Ansprüche 1 bis 8, **dadurch gekennzeichnet, daß** als Wirkstoff PVP-JOD enthalten ist.

10. Formkörper nach einem der Ansprüche 1 bis 9, **dadurch gekennzeichnet, daß** als Wirkstoff Metronidazol enthalten ist.

11. Formkörper nach einem der Ansprüche 1 bis 10, **dadurch gekennzeichnet, daß** der Formkörper ein zylindrischer, kegelförmiger oder walzenförmiger Stift ist.

12. Formkörper nach einem der Ansprüche 1 bis 11, **dadurch gekennzeichnet, daß** der Formkörper eine Zäpfchen- oder Torpedoform oder eine Tablettenform aufweist.

13. Verfahren zur Herstellung eines Formkörpers zur medizinischen Anwendung in Körper- und/oder Wundhöhlungen am menschlichen oder tierischen Körper, insbesondere nach einem der Ansprüche 1 bis 12, **dadurch gekennzeichnet**,
a) daß wenigstens eine hydrophile äußere Phase, beispielsweise ein wasserlösliches oder in Wasser dispergierbares Polymer, mit wenigstens einer lipophilen Komponente als innerer Phase zu einer Schmelze aufgeschmolzen wird, wobei die Schmelze mit wenigstens einem Wirkstoff oder einer Wirkstoffkombination versetzt und unter konstanten Temperaturbedingungen mechanisch zu einer Masse dispergiert wird,
b) daß die Masse in einen Behälter, ein Gefäß o.dgl. ausgegossen, darin mit einem konstanten Temperaturgradienten von wenigstens 5°C/min abgekühlt und anschließend mindestens 4 Stunden bei konstanter Temperatur zwischengelagert wird und
c) daß die dadurch gewonnene Masse auf eine Temperatur, die 5 bis 10 °C unterhalb des Erweichungspunktes liegt, erwärmt, durch Druck oder Scherung plastisch fließend gemacht und zu einem vorgebbaren Formkörper verpreßt wird, wobei der Arbeitsdruck 50 bar nicht unterschreitet, beispielsweise mindestens 100 bar beträgt, und daß die Arbeitstemperatur unterhalb der Erweichungstemperatur der Masse liegt.

14. Verfahren nach Anspruch 13 **dadurch gekennzeichnet, daß** der bzw. die Wirkstoffe oder die Wirkstoffkombination einem aufgeschmolzenen, niedrig-molekularen Polymer zugesetzt wird, wobei diese Vormischung dispergiert und der Schmelze beigemischt wird.

15. Verfahren nach einem der Ansprüche 13 oder 14, **dadurch gekennzeichnet, daß** die Masse bei konstanter Temperatur und unter definierbarem Arbeitsdruck zu fest-dispersen Formkörpem verpreßt wird.

16. Verfahren nach einem der Ansprüche 13 bis 15, **dadurch gekennzeichnet, daß** die Masse in einer Strangpresse zu Strängen von 2 bis 19 mm Durchmesser, vorzugsweise von 2 bis 10 mm Durchmesser, verpreßt wird, die zu Längen von 40 bis 100 mm zerteilt werden.

17. Verfahren zur Herstellung von gepreßten Formkörpem, welche einen hohen Gehalt an niedrig schmelzenden hydrophoben Bestandteilen besitzen, sowie zur Herstellung von fließfähigen oder rieselfähigen tablettierbaren Mischungen, welche einen hohen Gehalt an niedrig schmelzenden hydrophoben Bestandteilen aufweisen, nach einem der Ansprüche 13 bis 16, **dadurch gekennzeichnet,**
a) daß kleine Perlen einer abgekühlten Schmelze verpreßt werden.
b) daß kleine Kügelchen pulverisiert und verpreßt werden.
c) daß ein abgekühltes Pulver in Kapseln verbracht wird.

18. Pharmazeutische Zubereitung, **gekennzeichnet durch** eine fest-disperse Fett-in-Polymer-Dispersion oder Fett-in-Detergens-Dispersion nach wenigstens einem der Ansprüche 1 bis 17.

19. Verwendung von PVP-Jod zusammen mit einem Poloxamer zur Herstellung eines bis 100 mm langen und bis 2 mm dünnen bruchstabilen und biegeelastischen Formkörpers zur Behandlung von Wunden in milchproduzierenden Organen landwirtschaftlicher Nutztiere.

## Claims

1. Shaped body for medical administration in body cavities and/or wound cavities in humans or animals, comprising at least one amphiphilic outer phase, for example, a water-dispersible polymer, and at least one lipophilic component as inner phase and also comprising at least one pharmaceutically effective water-soluble or fat-soluble material, the outer phase being a biologically neutral oligopolymer of a polyether, for example, a copolymer, **wherein**
a) the outer phase is a poloxamer such as poloxamer 124, poloxamer 188, poloxamer 237, poloxamer 338, or poloxamer 407,
b) the proportion of outer phase is at least 40 wt% and the proportion of inner phase is up to 50 wt%,
c) the outer phase, the inner phase and the pharmaceutically effective material being shaped, upon maturing or storage at constant temperature with liquefaction under compression, as a solid-dispersed composition,
d) the shaped body having a diameter of down to 2 mm and a length of up to 100 mm.

2. Shaped body as defined in claim 1, **wherein** the lipophilic component is a wax, particularly an ester of a long-chain fatty acid with a long-chain fatty alcohol, for example, officinal beeswax EuAB 98 or bleached beeswax EuAB 98, or that the lipophilic component is a fatty alcohol or a mixture of fatty alcohols, for example, stearyl alcohol or cetyl/stearyl alcohol.

3. Shaped body as defined in claim 1 or claim 2, **wherein** the lipophilic component is a triglyceride mixture, for example, higher-melting suppository compositions having a melting point above 40 °C.

4. Shaped body as defined in any one of claims 1 to 3, **wherein** the lipophilic component is a long-chain hydrocarbon mixture, for example, of higher-melting paraffin compositions having a melting point above 40°C.

5. Shaped body as defined in any one of claims 1 to 4, **wherein** the proportion of the lipophilic component is between 25 and 50 wt%, preferably between 40 and 50 wt%, and that the proportion of the outer phase is 40 to 70 wt%, preferably between 48 and 52 wt%.

6. Shaped body as defined in any one of claims 1 to 5, **wherein** the proportion of active substances or the combination of active substances does not exceed 10 wt%.

7. Shaped body as defined in any one of claims 1 to 6, **wherein** the active substance comprises an antibiotic agent, e.g., benzyl penicillin procaine or gentamycin, an antiseptic agent, an antimycotic agent and/or a virustatic agent, e.g., aciclovir.

8. Shaped body as defined in any one of claims 1 to 7, **wherein** the active substance comprises a glucocorticoid, e.g., a hydrocortisone, an ester thereof, a betamethason, an ester thereof or a triamcinolone acetonide.

9. Shaped body as defined in any one of claims 1 to 8, **wherein** the active substance comprises PVP iodine.

10. Shaped body as defined in any one of claims 1 to 9, **wherein** the active substance comprises metronidazol.

11. Shaped body as defined in any one of claims 1 to 10, **wherein** the shaped body is a cylindrical or a conical or a barrel-shaped stick.

12. Shaped body as defined in any one of claims 1 to 11, **wherein** the shaped body has the shape of a suppository or torpedo or is in the form of a tablet.

13. Process for the manufacture of a shaped body for medical administration in body cavities and/or wound cavities in humans or animals, particularly as defined in any one of claims 1 to 12, **wherein**
a) at least one hydrophiiic outer phase, for example, a water-soluble or water-dispersible polymer, is fused with at least one lipophilic component as inner phase to form a melt, to which at least one active substance or a combination of active substances is added, the whole being mechanically dispersed under constant temperature conditions to produce a composition,
b) said composition is poured into a receptacle, a vessel or the like where it is cooled at a constant cooling rate of at least 5 °C/min, after which it is stored for at least 4 hours at constant temperature, and
c) the composition thus obtained is heated to a temperature which is from 5 to 10 °C below its softening point, is rendered flowable by the application of pressure or shear forces and is compressed to a predefined shaped body, during which operation the operating pressure is not below 50 bar, amounting to e.g. at least 100 bar, and the operating temperature is below the softening point of the composition.

14. Process as defined in claim 13, **wherein** the active substance(s) or combination of active substances is added to a low-molecular molten polymer, which premix is dispersed and added to the melt.

15. Process as defined in claim 13 or claim 14, **wherein** the composition is compressed at constant temperature and under a specific operating pressure to form a solid-dispersed shaped body.

16. Process as defined in any one of claims 13 to 15, **wherein** the composition is compressed in an extruder to form extrudates of from 2 to 19 mm in diameter, preferably from 2 to 10 mm in diameter, and is cut up into lengths between 40 and 100 mm.

17. Process for the manufacture of compressed shaped bodies having a high content of low-melting hydrophobic components, as well as for the manufacture of flowable or pourable pelletizable mixtures having a high content of low-melting hydrophobic components, as defined in any one of claims 13 to 16, **wherein**
a) small beads of a cooled melt are compressed,
b) small spherules are pulverized and compressed,
c) a cooled powder is filled into capsules.

18. Pharmaceutical preparation, **characterized by** a dispersed solid fat-in-polymer dispersion or fat-in-detergent dispersion as defined in at least one of claims 1 to 17.

19. Use of PVP iodine together with a poloxamer for the production of a fracture-stable shaped body possessing flexural elasticity and having a length of up to 100 mm and a diameter of down to 2 mm for the treatment of wounds in milk-producing organs of agricultural working animals.

## Revendications

1. Corps moulé pour l'application médicale dans des cavités du corps et/ou des plaies des êtres humains et des animaux, contenant au moins une phase externe amphiphile, par exemple un polymère dispersible dans l'eau, et au moins un composant lipophile en tant que phase interne, ainsi qu'au moins une substance pharmaceutiquement active et soluble dans l'eau ou dans les graisses, la phase externe étant un oligopolymère biologiquement neutre d'un polyéther, par exemple un copolymère, **caractérisé par le fait**
a) que la phase externe est un poloxamère, par exemple poloxamère 124, poloxamère 188, poloxamère 237, poloxamère 338 ou poloxamère 407,
b) que la proportion de la phase externe est au moins 40 % en poids et la proportion de la phase interne est jusqu'à 50 % en poids,
c) que la phase externe, la phase interne et la substance pharmaceutiquement active composent une masse solide-dispersée qui est moulée après une période définie de maturation ou de stockage à température constante par liquéfaction sous pression,
d) et que le corps moulé a un diamètre minimum de 2 mm et une longueur maximum de 100 mm.

2. Corps moulé selon la revendication 1, **caractérisé par le fait que** le composant lipophile est une cire, notamment un ester d'acide gras à longue chaîne avec de l'alcool gras à longue chaîne, par exemple cire d'abeille officinale EuAB 98 ou cire d'abeille blanche EuAB 98, ou que le composant lipophile est un alcool gras ou un mélange d'alcools gras, par exemple alcool stéarylique ou alcool cétylique/stéarylique.

3. Corps moulé selon la revendication 1 ou 2, **caractérisé par le fait que** le composant lipophile est un mélange de triglycérides, par exemple des compositions de suppositoires à point de fusion élevé, à savoir supérieur à 40°C.

4. Corps moulé selon l'une des revendications 1 à 3, **caractérisé par le fait que** le composant lipophile est un mélange de carbures d'hydrogènes à longue chaîne, par exemple des compositions de paraffine à point de fusion élevé, à savoir supérieur à 40 °C.

5. Corps moulé selon l'une des revendications 1 à 4, **caractérisé par le fait que** la proportion du composant lipophile est entre 25 et 50 % en poids, de préférence entre 40 et 50 % en poids, et que la proportion de la phase externe est entre 40 et 70 % en poids, de préférence entre 48 et 52 % en poids.

6. Corps moulé selon l'une des revendications 1 à 5, **caractérisé par le fait que** la proportion des substances actives ou de la combinaison des substances actives ne dépasse pas 10 % en poids.

7. Corps moulé selon l'une des revendications 1 à 6, **caractérisé par le fait que** la substance active contient un antibiotique, p.ex. benzylpenicilline procaïne ou gentamicine, un antiseptique, une substance antimycosique et/ou une substance virustatique, p.ex. aciclovir.

8. Corps moulé selon l'une des revendications 1 à 7, **caractérisé par le fait que** la substance active contient un glucocorticoïde, p.ex., une hydrocortisone, son ester, une bétaméthasone, son ester ou une triamcinolone acétonide.

9. Corps moulé selon l'une des revendications 1 à 8, **caractérisé par le fait que** la substance active contient PVP-iode.

10. Corps moulé selon l'une des revendications 1 à 9 , **caractérisé par le fait que** la substance active contient métronidazole.

11. Corps moulé selon l'une des revendications 1 à 10, **caractérisé par le fait que** le corps moulé est un petit bâton cylindrique, conique ou en forme de barrique.

12. Corps moulé selon l'une des revendications 1 à 11, **caractérisé par le fait que** le corps moulé est en forme de suppositoire, de torpille ou de tablette.

13. Procédé de fabrication des corps moulés pour l'application médicale dans des cavités du corps et/ou des plaies des êtres humains ou des animaux, notamment selon l'une quelconque des revendications 1 à 12, **caractérisé par le fait**
a) qu'au moins une phase externe hydrophile, p. ex. un polymère soluble ou dispersible dans l'eau, est mise en fusion avec au moins un composant lipophile en tant que phase interne pour obtenir ainsi une masse fondue, tout en y ajoutant au moins une substance active ou une combinaison de substances actives et en dispersant la masse fondue mécaniquement à température constante pour obtenir une masse à traiter,
b) que ladite masse est versée dans un récipient, un réservoir ou similaire, refroidie à un gradient de température constant d'au moins 5 °C/min et ensuite stockée à température constante pour au moins 4 heures et
c) que la masse ainsi obtenue est chauffée à une température inférieure de 5 à 10 °C à son point de ramollissement, rendue fluide et déformée par compression ou cisaillement pour obtenir ainsi un corps moulé défini, la pression de travail ne restant pas inférieure à 50 bar, p.ex. s'élevant au minimum à 100 bar, et la température de travail ne dépassant pas la température de ramollissement de la masse.

14. Procédé selon la revendication 13, **caractérisé par le fait que** la substance active (ou les substances actives) ou la combinaison de substances actives est ajoutée à un polymère fondu à basse masse moléculaire et que ce prémélange est dispersé et ajouté à la masse fondue.

15. Procédé selon la revendication 13 ou 14, **caractérisé par le fait que** la masse est comprimée à température constante et sous pression de travail définie pour obtenir ainsi des corps moulés solides-dispersés .

16. Procédé selon l'une des revendications 13 à 15, **caractérisée par le fait que** la masse est introduite dans une extrudeuse qui produit par compression des barres d'un diamètre de 2 à 19 mm, de préférence de 2 à 10 mm, qui sont ensuite coupées en pièces d'une longueur de 40 à 100 mm.

17. Procédé de fabrication des corps moulés comprimés ayant un taux élevé de composants hydrophobes à bas point de fusion ainsi que pour produire des mélanges **caractérisés par** leur fluidité ou faculté d'écoulement et leur aptitude à la compression ayant un taux élevé de composants hydrophobes à bas point de fusion, selon l'une quelconque des revendications 13 à 16, **caractérisé par le fait que**
a) de petites perles d'une masse fondue refroidie sont comprimées,
b) de petits globules sont réduits en poudre et comprimés,
c) une poudre est refroidie et mise dans des capsules.

18. Préparation pharmaceutique, **caractérisée par** une dispersion solide dispersée de la graisse dans des polymères ou une dispersion de la graisse dans des détergents selon au moins une des revendications 1 à 17.

19. Utilisation de PVP-iode avec un poloxamère pour produire un corps moulé résistant à la rupture et flexible, ayant une longueur maximum de 100 mm et un diamètre minimum de 2 mm pour traiter des plaies dans les organes produisant du lait d'animaux domestiques agricoles.
